# EUROPÄISCHE PATENTSCHRIFT

(11) **EP 4 599 232 B1**
(45) Veröffentlichungstag und Bekanntmachung des Hinweises auf die Patenterteilung: **19.08.2026**
(21) Anmeldenummer: 23834113.5
(22) Anmeldetag: 19.12.2023
(51) Int. Cl.: G01N 21/64, G01N 33/50, G01N 33/92, G01N 33/543, G01N 21/77, G01N 15/0205

(54) **VERFAHREN ZUR IN-SITU ERFASSUNG VON ÄNDERUNGEN EINES LIPIDSYSTEMS BEI DESSEN LAGERUNG BEI EINER LAGERTEMPERATUR UNTERHALB VON -60 °C**
METHOD FOR IN-SITU DETECTION OF CHANGES IN A LIPID SYSTEM DURING THE STORAGE THEREOF AT A STORAGE TEMPERATURE OF BELOW -60°C
PROCÉDÉ DE DÉTECTION IN SITU DE CHANGEMENTS DANS UN SYSTÈME LIPIDIQUE PENDANT SON STOCKAGE À UNE TEMPÉRATURE INFÉRIEURE À -60 °C

(30) Priorität: 20.12.2022 DE 102022134188
(43) Veröffentlichungstag der Anmeldung: 13.08.2025
(73) Patentinhaber: Universität Augsburg - Körperschaft des öffentlichen Rechts, 86159 Augsburg (DE)
(72) Erfinder: WESTERHAUSEN, Christoph, 86179 Augsburg (DE); FÄRBER, Nicolas, 86159 Augsburg (DE)
(74) Vertreter: Charrier Rapp & Liebau
(86) Internationale Anmeldenummer: PCT/EP2023/086532
(87) Internationale Veröffentlichungsnummer: WO 2024/133193

(56) Entgegenhaltungen:
- PAKUNLU R I ET AL: "In vitro and in vivo intracellular liposomal delivery of antisense oligonucleotides and anticancer drug", JOURNAL OF CONTROLLED RELEASE, ELSEVIER, AMSTERDAM, NL, vol. 114, no. 2, 28 August 2006 (2006-08-28), pages 153 - 162, XP024957586, ISSN: 0168-3659, [retrieved on 20060828], DOI: 10.1016/J.JCONREL.2006.06.010
- HAN SUK KYU ET AL: "Oleanolic acid and ursolic acid stabilize liposomal membranes", LIPIDS, vol. 32, no. 7, 1 July 1997 (1997-07-01), DE, pages 769 - 773, XP093138465, ISSN: 0024-4201, DOI: 10.1007/s11745-997-0098-9
- AHN-YOON SOOHYOUN ET AL: "Ganglioside?liposome immunoassay for the detection of botulinum toxin", ANALYTICAL AND BIOANALYTICAL CHEMISTRY, vol. 378, no. 1, 1 January 2004 (2004-01-01), Berlin/Heidelberg, pages 68 - 75, XP055808590, ISSN: 1618-2642, Retrieved from the Internet <URL:https://link.springer.com/content/pdf/10.1007/s00216-003-2365-4.pdf> DOI: 10.1007/s00216-003-2365-4
- BURKS S R ET AL: "Cellular uptake of electron paramagnetic resonance imaging probes through endocytosis of liposomes", BIOCHIMICA ET BIOPHYSICA ACTA, ELSEVIER, AMSTERDAM, NL, vol. 1788, no. 10, 1 October 2009 (2009-10-01), pages 2301 - 2308, XP026684881, ISSN: 0005-2736, [retrieved on 20090825], DOI: 10.1016/J.BBAMEM.2009.08.007

## Beschreibung

Die Erfindung betrifft ein Verfahren zur in-situ Erfassung von Änderungen der Struktur und/oder der Komposition und/oder der Hydrierung eines Lipidsystems bei dessen Lagerung bei einer Lagertemperatur unterhalb von -60 °C nach Anspruch 1.

Lipidsysteme stehen beispielsweise als Träger von Pharmazeutika (lipidgestütze Medikamente), wie beispielsweise Lipidnanopartikel als Träger moderner mRNA-Impfstoffe, oder bei der Behandlung von Tumoren in Form von sog. Liposomen im Fokus aktueller pharmazeutischer Forschung. Neben solchen synthetischen Formen bilden Lipidsysteme das Grundgerüst von Zellmembranen und steuern wichtige Zellfunktionen, wie beispielsweise deren Permeabilität und elastische Verformbarkeit, weshalb sie ebenfalls in der Biologie und Biophysik von zentraler Bedeutung sind.

Im Folgenden sind unter einem Lipidsystem in Wasser oder sonstigen polaren oder apolaren Lösemitteln eingelagerte Lipide, die insbesondere kolloidale Strukturen (Größenordnung: 10 bis 1000 nm), speziell Lipidnanopartikel (Größenordnung < 100 nm), Lipidmikropartikel (Größenordnung: 100 nm bis 100 µm), Liposome, Vesikel oder Zellmembranen etc. bilden, zu verstehen. Die Lipide können dabei insbesondere Träger eines Pharmazeutikums, insbesondere von (nukleinsäurebasierten) Wirk- oder Impfstoffen, oder Träger von DNA, RNA oder Proteinen sein. Bei den Lösemitteln kann es sich beispielsweise um Wasser, wässrige Puffer, Ethanol, Methanol, Glycerol, Butanol, Cyclohexan, Dietehylether, etc. handeln.

Insbesondere im pharmazeutischen Bereich wirken sich Änderungen verwendeter Lipidsysteme, wie beispielsweise Änderungen der Struktur oder der Komposition oder der Hydrierung des Lipidsystems, maßgeblichen auf deren Eigenschaften und infolgedessen auf den Zustand und die Wirkung der von diesen getragenen Pharmazeutika aus. Beispielsweise kann eine Strukturänderung in einem Lipidnanopartikel zu einer Schädigung der eingeschlossenen mRNA führen. Üblicherweise sollen derartige Änderungen der Lipidsysteme durch Lagerung bei tiefen Temperaruten, d.h. insbesondere bei Temperaturen unterhalb von - 60°C oder -80°C oder noch tieferen Temperaturen, bei denen sich das Lipidsystem in einem gefrorenen Zustand befindet, verhindert oder zumindest in gewissen Grenzen gehalten werden. Herkömmlicherweise werden lagerungsbedingte Änderungen eines Lipidsystems durch kalorimetrische Messungen oder durch dynamische Lichtstreuung erfasst. Diese Messungen erfolgen dabei, insbesondere auch aufgrund von Beschränkungen die diese herkömmlichen Messverfahren mit sich bringen, nach erfolgter Lagerung, an dem, insbesondere auf Temperaturen oberhalb von 0°C, erwärmten flüssigen Lipidsystem. Insbesondere die Ermittlung geeigneter Lagerbedingungen, bei der eine Änderung des Lipidsystems innerhalb akzeptabler Grenzen bleibt, wie die Ermittlung einer geeigneten Lagertemperatur bzw. die maximal mögliche Lagerdauer bei einer bestimmten Lagertemperatur, ist bei den herkömmlichen Messverfahren aufwendig und zeitintensiv. Insbesondere ist das Ergebnis auch erst nach der erfolgten Lagerung zugänglich.

Aus wissenschaftlichen Veröffentlichungen, beispielsweise aus den Veröffentlichungen PAKUNLU R. I. ET AL: "In vitro and in vivo intracellular liposomal delivery of antisense oligonucleotides and anticancer drug", Journal of controlled release, Elsevier, Amsterdam, NL, Bd. 114, Nr. 2, 28. August 2006, Seiten 153-162 ISSN: 0168-3659 oder Han suk Kye ET AL: "Oleanolic acid and ursolic acid stabilize liposomal membranes", Lipids, Bd. 32, Nr. 7, 1. Juli 1997, Seiten 769 - 773, ISSN: 0024-4201 oder Ahn-Yoon Soohyoun ET AL: "Gangliosideliposome immunoassay for the detection of botulinum toxin", Analytical and Bioanalytical Chemistry, Bd. 378, Nr. 1, 1. Januar 2004, Seiten 68-75, ISSN: 1618-2642 oder Burks S. R. ET AL: "Cellular uptake of electron paramagnetic resonance imaging probes through endocytosis of liposomes", Biochimica et Biophysica Acta, Elsevier, Amsterdam, NL, Bd. 1788, Nr. 10, 1.Oktober 2009, Seiten 2301-2308, ISSN: 0005-2736, sind zudem Fluoreszenzmessungen eines erwärmten Lipidsystems bzw. eines Lipidsystems bei Temperaturen oberhalb von 0 °C im Allgemeinen bekannt.

Es besteht daher die Aufgabe, ein Verfahren bereitzustellen, dass die Erfassung von Änderungen eines Lipidsystems, die im Rahmen von dessen Lagerung auftreten, auf vorteilhafte Weise ermöglicht.

Gelöst wird diese Aufgabe mit einem Verfahren nach Anspruch 1. Vorteilhafte Ausgestaltungen und zweckmäßige Weiterbildungen der Erfindung sind den Unteransprüchen entnehmbar.

Durch das erfindungsgemäße Verfahren ist eine in-situ Erfassung von Änderungen der Struktur und/oder der Komposition und/oder der Hydrierung eines Lipidsystems bei bzw. im Rahmen von dessen Lagerung bei Lagertemperaturen unterhalb von -60 °C, d.h. speziell unmittelbar während der Lagerung bei Lagertemperaturen unterhalb von -60 °C, möglich. Die Lagerung des Lipidsystems bei Lagertemperaturen unterhalb von -60°C ist dabei insbesondere eine Lagerung über mehrere Stunden oder über mehrere Tage. Unter der in-situ Erfassung ist eine Messung bzw. die Erfassung zu verstehen, die erfolgt, während die (lagerbedingte) Änderung des Lipidsystems stattfindet. Bei der in-situ Erfassung handelt es sich folglich um die unmittelbare Erfassung von Änderungen eines Lipidsystems während der Lagerung bei einer Lagertemperatur unterhalb von -60°C und nicht um eine nachgelagerte Messung an einer auf höhere Temperaturen erwärmten, insbesondere aufgetauten, Probe. Unter der Änderung der Struktur des Lipidsystems sind dabei insbesondere Änderungen der molekularen Ordnung, die Größenänderung von Partikeln, eine Aggregation oder ein Kompaktieren des Lipidsystems, die Entmischung der Lipidlegierung innerhalb der Partikel oder auch in verschiedenen Partikelpopulationen, die Änderung der Einlagerung des Wirkstoffs in dem Lipidsystem oder der Verlust eines in dem Lipidsystem eingelagerten Wirkstoffs, etc. zu verstehen. Strukturänderungen des Lipidsystems können dabei insbesondere auch durch strukturelle Änderungen des getragenen Pharmazeutikums bedingt sein. Unter der Änderung der Komposition ist insbesondere die Dekomposition bzw. ein Verlust einzelner Bestandteile von Lipidstrukturen bzw. Lipidanordnungen, wie beispielsweise der Verlust einzelner Bestandteile eines Lipidnanopartikels, etc., des Lipidsystems oder die Oxidation einzelner Moleküle des Lipidsystems zu verstehen. Unter einer Änderung der Hydrierung ist die Änderung der Hydrierung der Lipidstrukturen des Lipidsystems zu verstehen.

Für das erfindungsgemäße Verfahren wird das zu untersuchende Lipidsystem, insbesondere vorab bzw. in einem vorgelagerten Schritt, mit zumindest einem fluoreszierenden Farbstoff versehen. Speziell handelt es sich dabei um einen fluoreszierenden Farbstoff, der mit dem zu untersuchenden Lipidsystem wechselwirkt. Der fluoreszierende Farbstoff kann dabei insbesondere in Strukturen des Lipidsystems eingebunden sein. Beispielsweise kann es sich bei dem fluoreszierenden Farbstoff um Laurdan (C₂₄H₃₅NO), Prodan (C₁₅H₁₇NO), C-Laurdan (C₂₅H₃₅NO₃), Pro12A (C₃₁H₄₇N₂O₅SNa₂), Di-4-ANEPPDHQ (C₃₂H₄₇Br₂N₃O₂) oder Atto 488 mit verschiedenen Lipidankernen oder einer Kombination dieser handeln. Es ist aber auch ein anderer Farbstoff denkbar, der in ähnlicher Weise wie die hier explizit genannten Farbstoffe sensitiv mit dem zu untersuchenden Lipidsystem wechselwirkt. Das Einbringen des Farbstoffs kann dabei, insbesondere auch in Abhängigkeit von dem zu untersuchenden Lipidsystem bzw. dem zu verwendenden Farbstoff, auf verschiedene Weise erfolgen, beispielsweise durch Zugabe zu den Lipiden des Lipidsystems bei der Synthese oder der Formulierung (z.B. im Fall von Lipidnanopartikeln oder Liposomen) oder durch Zugabe zu dem Lösemittel des suspendierten Lipidsystems. Unter anderem kann der Farbstoff auch zunächst in einem ersten Lösemittel gelöst werden und anschließend zu dem Lipidsystem bzw. dem bei dem Lipidsystem verwendeten zweiten Lösemittel hinzugegeben werden.

Weiter umfasst das erfindungsgemäße Verfahren die folgenden Schritte:
- Anregen des fluoreszierenden Farbstoffs des Lipidsystems mit Licht, insbesondere durch eine Lichtquelle, die Licht im Absorptionsbereich des fluoreszierenden Farbstoffs emittiert, insbesondere Licht im UV-Bereich (speziell im Bereich 100 nm bis 390 nm) oder im sichtbaren Bereich (390 nm bis 780 nm) oder im IR-Bereich (speziell im Bereich 780 nm bis 1400 nm);
- Erfassen eines von dem fluoreszierenden Farbstoff des Lipidsystems infolge der Anregung ausgesendeten Fluoreszenzspektrums bzw. zumindest eines Teils des Fluoreszenzspektrums mit einem Detektor, insbesondere einem Spektrometer;
- Vergleichen von zumindest einem Teil des erfassten Fluoreszenzspektrums mit einem entsprechenden Teil eines Referenzspektrums; und
- Erfassen von Abweichungen gegenüber dem Referenzspektrum, die ein Maß für die Änderung des Lipidsystems sind.

Der Einsatz der Fluoreszenzspektroskopie an Lipidsystemen, speziell ausgewählten (Zell-) Membranen und Lipidvesikeln, mit darin eingebettetem Farbstoff ist aus der Veröffentlichung "Broad lipid phase transitions in mammalian cell membranes measured by Laurdan fluorescence spectroscopy" in Biochemica et Biophysica Acta (BBA) - Biomembranes 1864 (2022), 183794 der Erfinder im Allgemeinen bekannt. Bei der genannten Studie wurden Phasenübergänge des Ordnungszustands von speziellen Lipidsystemen sowie die Abhängigkeit des Phasenübergangs vom Cholesteringehalt, dem pH-Wert und Anästhetikum durch Messungen bei Temperaturen zwischen -40°C bis +90°C untersucht. Speziell wurden dabei die Änderungen des Fluoreszenzspektrums in Abhängigkeit der Temperatur untersucht. Aus den erhaltenen Messwerten konnte der Temperaturbereich des Phasenübergangs bestimmt werden. Die erfassten Messwerte der signifikanten Änderungen im Bereich des Phasen-Übergangs lieferten, beispielsweise durch die Breite und Höhe des Phasen-Übergang-Peaks, weitere Informationen zu dem Phasenübergang.

Die Ergebnisse aus der genannten Veröffentlichung betreffend die Untersuchung des Phasen-Übergangs deuten darauf hin, dass unterhalb von -40°C keine Änderungen des dabei betrachteten Parameters bzw. keine Änderungen des Fluoreszenzspektrums erfolgen bzw. messbar sind. Im Rahmen der vorliegenden Erfindung wurde festgestellt, dass auch bei sehr tiefen Temperaturen, d.h. insbesondere Temperaturen (deutlich) kleiner als -60°C oder -80°C, beispielsweise Temperaturen unterhalb von -100°C oder auch Temperaturen bis -196°C, Dynamiken eines fluoreszierenden Farbstoffs eines Lipidsystems auftreten. Weiter wurde festgestellt, dass diese im Vergleich zu den signifikanten Änderungen im Bereich des Phasenübergangs äußerst geringen Änderungen des Fluoreszenzspektrums auch erfassbar sind und für die Erfassung von Änderungen des Lipidsystems bei dessen Lagerung herangezogen werden können.

Das erfindungsgemäße Verfahren bietet gegenüber herkömmlichen Verfahren zur Erfassung lagerbedingter Änderungen des Lipidsystems, wie beispielsweise kalorimetrischen Messungen, den Vorteil, dass bereits unmittelbar während des Lagerprozesses Änderungen des Lipidsystems bzw. die Stabilität des Lipidsystems in Abhängigkeit bestimmter Lagerbedingungen, wie beispielsweise der Lagertemperatur und der Lagerzeit, erfasst werden können. Bei dem erfindungsgemäßen Verfahren werden insbesondere Änderungen eines Lipidsystems nicht erst nach einem der Lagerung nachgelagerten Auftauen sondern bereits unmittelbar während der Lagerung bei Lagertemperaturen unterhalb von -60 °C erfasst, d.h. insbesondere Änderungen eines sich in gefrorenem Zustand befindenden Lipidsystems. Hierdurch ist es beispielsweise auch möglich, die Lagerbedingungen unmittelbar während der Lagerung anzupassen, beispielsweise durch Änderung der Lagertemperatur. Weiter ist auch unmittelbar detektierbar, zu welchem Zeitpunkt Änderungen auftreten, so dass unmittelbar Rückschlüsse auf eine geeignete bzw. maximale Lagerzeit in Abhängigkeit der gewählten Lagertemperatur möglich sind. Durch das erfindungsgemäße Verfahren können daher auf vorteilhafte Weise geeignete Lagerbedingungen für Lipidsysteme ermittelt und bestimmt werden.

In einer bevorzugten Ausgestaltung umfasst das Verfahren einen Schritt des Erfassens und/oder Hinterlegens eines Referenzspektrums. Insbesondere können auch mehrere Referenzspektren erfasst und hinterlegt werden, wodurch ein Vergleich des erfassten Fluoreszenzspektrums des zu untersuchenden Lipidsystems mit mehreren Referenzspektren möglich ist. Das Referenzspektrum kann dabei insbesondere ein erfasstes Fluoreszenzspektrum des Lipidsystems zu einem früheren Zeitpunkt der Lagerung, speziell zu einem Ausgangszeitpunkt bzw. zum Beginn der Lagerung sein. Hierdurch ist es möglich, die an dem untersuchten Lipidsystem auftretenden Änderungen bzw. den zeitlichen Verlauf der Änderungen unmittelbar zu beobachten und zu vergleichen. Weiter oder ergänzend hierzu kann das Referenzspektrum auch das Fluoreszenzspektrum eines in einer Datenbank gespeicherten Referenzsystems sein. Insbesondere können auch mehrere Referenzspektren unterschiedlicher Referenzsysteme und/oder eines Referenzsystems bei unterschiedlichen Lagerbedingungen hinterlegt sein. Durch den Vergleich mit den Referenzspektren verschiedener Referenzsysteme bzw. Referenzspektren eines Referenzsystems bei unterschiedlichen Lagerbedingungen ist beispielsweise eine (bessere) Einordnung und Charakterisierung von Änderungen möglich.

Der Vergleich des erfassten Fluoreszenzspektrums mit dem Referenzspektrum und infolgedessen auch das zugehörige Erfassen der Abweichungen kann auf Basis der jeweils erfassten Intensitätswerte der Spektren oder eines Teils dieser Spektren, beispielsweise der Intensitätswerte einer oder mehrerer bestimmter Wellenlängen bzw. Wellenlängenbereiche, erfolgen. In einer vorteilhaften Ausgestaltung des Verfahrens erfolgt das Vergleichen zumindest eines Teils des erfassten Fluoreszenzspektrums mit einem entsprechenden Teil eines Referenzspektrums auf Basis von ausgewählten Parametern, die aus dem Fluoreszenzspektrum und dem Referenzspektrum jeweils berechnet werden bzw. im Falle des Referenzspektrums bereits berechnet sind. Beispielsweise kann es sich hierbei um Intensitätsverhältnisse bestimmter Teile der Spektren, beispielsweise Intensitätsverhältnisse bestimmter Wellenlängen bzw. Wellenlängenbereiche, handeln. Alternativ können auch andere aus den Spektren ableitbare Parameter, wie eine "Welligkeit" des Spektrums oder eine besondere Krümmung oder ähnliches für den Vergleich herangezogen werden. Die für den Vergleich verwendeten Charakteristika können insbesondere durch analytische, numerische oder KI-Verfahren (Erkennen bestimmter Muster) ermittelt werden. In einer vorteilhaften Ausgestaltung ist es beispielsweise auch denkbar, dass eine bestimmte Polarisation (bzw. Lichtpolarität) des von dem fluoreszierenden Farbstoff ausgesendeten Fluoreszenzspektrums, insbesondere unter Verwendung von für die Erfassung dieser Polarisation entsprechend angeordneten Polfiltern, erfasst bzw. mit einem entsprechenden Wert des Referenzspektrums, speziell einer hinterlegten oder erfassten Polarisation des Referenzspektrums, verglichen wird. Durch die genannten Ausgestaltungen ist ein vorteilhafter, insbesondere auch automatisierbarer Vergleich der Spektren gegeben, der die Erfassung der Änderungen des Lipidsystems erleichtert.

In einer vorteilhaften Ausgestaltung des Verfahrens erfolgt eine Erfassung der Änderungen des Lipidsystems in Abhängigkeit der Lagerzeit und/oder der Lagertemperatur. Eine Erfassung in Abhängigkeit der Lagertemperatur bedeutet hierbei eine Änderung der Lagertemperatur unterhalb von -60°C hin zu einer anderen Lagertemperatur unterhalb von -60°C, und eine Erfassung der Änderung bei Fahren auf diese neue Lagertemperatur und Halten dieser neuen Lagertemperatur. Bei einer Erfassung in Abhängigkeit der Lagerzeit werden beispielsweise zeitliche Änderungen von Intensitätswerten bestimmter Bereiche des erfassten Fluoreszenzspektrums oder die zeitliche Änderung von speziellen Intensitätsverhältnissen oder die zeitliche Änderung der Polarisation des von dem fluoreszierenden Farbstoff emittierten Fluoreszenzspektrums erfasst, die insbesondere Rückschlüsse auf eine (zeitliche) Änderung des Lipidsystems ermöglichen. Bevorzugt erfolgt dabei eine kontinuierliche zeitliche Erfassung der Änderungen des Lipidsystems. Vorzugsweise erfolgt ein Erfassen der von dem fluoreszierenden Farbstoff ausgesendeten Fluoreszenzspektren im Abstand von wenigen Mikrosekunden oder Millisekunden, beispielsweise im Bereich von einer Mikrosekunde bis 100 Millisekunden, so dass auch kurzzeitige Änderungen des Lipidsystems sowie der Verlauf von Änderungen möglichst genau erfassbar sind. Durch den hierdurch erfassten zeitlichen bzw. lagertemperaturbedingten Verlauf der Änderungen sind insbesondere Rückschlüsse auf geeignete bzw. optimale Lagerbedingungen möglich.

In einer weiteren Ausgestaltung des Verfahrens kann eine kurzzeitige Änderung der Lagertemperatur, beispielsweise auch eine kurzzeitige Erhöhung auf Temperaturen oberhalb von -60°C, und ein Erfassen der Änderung des Lipidsystems in Abhängigkeit dieser kurzzeitigen Änderung der Lagertemperatur erfolgen. Hierdurch kann beispielsweise das Verhalten des Lipidsystems bei einer Kühlkettenunterbrechung beobachtet werden bzw. eine Kühlkettenunterbrechung simuliert werden. Hierdurch sind insbesondere Rückschlüsse zu den Auswirkungen kurzzeitiger, beispielsweise transportbedingter Kühlkettenunterbrechungen möglich. Weiter können hierdurch auch Auswirkungen von Ausfällen in der Kühlkette analysiert werden.

In einer vorteilhaften Ausgestaltung umfasst das Verfahren das Erfassen der temperaturprofilabhängigen Änderung des Lipidsystems bei der anfänglichen Abkühlung des Lipidsystems, beispielsweise ausgehend von Raumtemperatur, auf die Lagertemperatur und/oder bei abschließender Erwärmung des Lipidsystems nach erfolgter Lagerung auf eine Gebrauchstemperatur, insbesondere bei Erwärmen auf eine Temperatur über 0°C. Hierdurch ist eine Erfassung und Abbildung der gesamten lagerungsbedingten Änderungen eines Lipidsystems beginnend mit der Einlagerung bzw. dem Einfrieren bis hin zum Auftauen und Gebrauch nach erfolgter Lagerung möglich.

Bevorzugt kann das Verfahren auch einen Schritt des Bestimmens von geeigneten Lagerbedingungen des Lipidsystems, insbesondere einer geeigneten Lagertemperatur und/oder Lagerzeit, umfassen. Geeignete Lagerbedingungen sind insbesondere solche, bei denen eine durch die Fluoreszenzspektroskopie erfassbare Änderung des Lipidsystems unterhalb eines vorgegebenen Grenzwerts liegt.

In einer weiteren Ausgestaltung kann die Ausgabe eines Warnsignals und/oder eines Warnhinweises erfolgen, wenn eine Abweichung des Fluoreszenzspektrums gegenüber dem Referenzspektrum erfasst wird, die über eine vorgegebene Grenzabweichung hinausgeht. Hierdurch ist beispielsweise das unmittelbare Erkennen und eine Aussonderung von degradierten, d.h. zu stark von Ihrer vorgesehenen bzw. ursprünglichen Struktur abweichenden Lipidsystemen, möglich. Weiter ist auch denkbar, dass das Warnsignal bzw. der Warnhinweis angibt, dass eine zeitnahe Verwendung eines einen Wirkstoff tragenden Lipidsystemen zu erfolgen hat, da dieser ansonsten unbrauchbar wird.

Weiter kann in einer bevorzugten Ausgestaltung auch ein Anpassen der Lagertemperatur unmittelbar während der Lagerung infolge einer erfassten Abweichung erfolgen. Beispielsweise kann eine weitere Absenkung der Lagertemperatur erfolgen, um einer erfassten, zu starken Änderungen des Lipidsystems entgegenzuwirken. Weiter kann beispielsweise aus energetischen Gründen auch ein Erhöhen der Lagertemperatur erfolgen, wenn keine oder nur eine sehr geringe Änderung des Lipidsystems erfasst wird.

In einer vorteilhaften Ausgestaltung kann das Verfahren auch einen Schritt des Bestimmens und/oder der Charakterisierung der Änderung der Struktur und/oder der Komposition und/oder der Hydrierung auf Basis der erfassten Abweichungen umfassen. Durch diese Zuordnung der Änderung sind insbesondere Rückschlüsse auf die Ursache der Änderung und ggf. wie diesen Änderungen entgegengewirkt werden kann, möglich. Beispielsweise ist es denkbar, dass einer bestimmten Änderung durch Zugabe eines Zuschlagsstoffs zu dem Lipidsystem vor der Lagerung gezielt entgegengewirkt werden kann.

Bevorzugt umfasst das Verfahren einen Schritt des Vergleichens einer erfassten Änderung des Lipidsystems mit der durch ein anderes Messverfahren erfassten Änderung. Beispielsweise kann hierbei ein Vergleich mit durch eine kalorimetrische Messung und/oder mittels dynamischer Lichtstreuung an dem Lipidsystem bei Temperaturen oberhalb von 0° C erfassten Änderung erfolgen. Durch die Kombination der Resultate der unterschiedlichen Messverfahren sind beispielsweise Vorteile für eine Zuordnung und Charakterisierung erfasster Änderungen möglich.

Diese und weitere Merkmale sowie Vorteile und Wirkungen des erfindungsgemäßen Verfahrens ergeben sich aus dem nachfolgenden unter Bezugnahme auf die begleitenden Zeichnungen näher beschriebenen Ausführungsbeispiel. Die Zeichnungen zeigen:
- **Fig. 1**: eine skizzenhafte Darstellung eines Messsystems in einer Schnittansicht;
- **Fig. 2**: ein erstes Diagramm einer beispielhaften Intensitätsmessung sowie deren Auswertung;
- **Fig. 3**: ein Diagramm eines aus einer Messung berechneten Parameters P in Abhängigkeit der Lagerzeit;
- **Fig. 4**: ein beispielhaftes Lipidsystem als Träger eines Wirkstoffs;
- **Fig. 5**: das Lipidsystem aus Fig. 4 mit geänderter Struktur;
- **Fig. 6**: ein beispielhaftes Lipidsystem aus zwei unterschiedlichen Lipiden als Träger eines Wirkstoffs;
- **Fig. 7**: das Lipidsystem aus Fig. 7 mit geänderter Komposition.

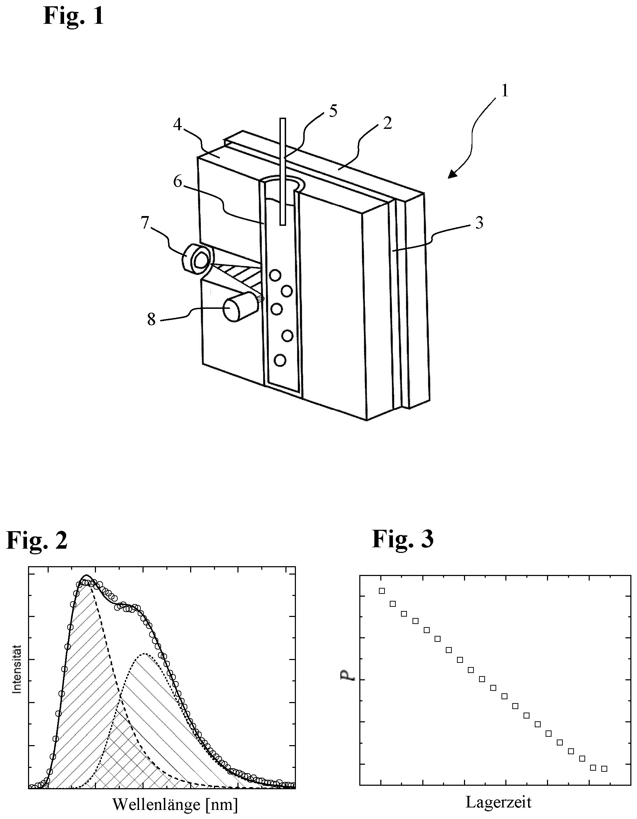

In Fig. 1 ist ein Schnitt durch ein skizziertes Ausführungsbeispiel einer Messvorrichtung 1 zur Durchführung des erfindungsgemäßen Verfahrens gezeigt. Die Messvorrichtung 1 umfasst eine äußere Einheit 2, beispielsweise einen wasser- oder luftgekühlten Metallkörper, insbesondere eine wassergekühlte Kupferschicht, eine daran anschließende mittlere Einheit 3, beispielsweise ein Peltier-Element, und eine innere Einheit 4, beispielsweise einen Metallblock, insbesondere einen Aluminiumblock. Die innere Einheit 4 weist dabei eine Aussparung auf, in die ein das zu untersuchende Lipidsystem enthaltendes Probengefäß 6, insbesondere eine Glaskapillare oder ein Reaktionsgefäß aus Kunststoff, eingebracht ist. Über die Einheiten 4, 5, 6 ist dabei eine Temperierung des zu untersuchenden Lipidsystems, insbesondere ein Abkühlen auf Temperaturen unterhalb von -60°C, möglich. Über einen in das Probengefäß 6 eingebrachten Temperatursensor 5 ist eine Erfassung der Temperatur des Lipidsystems möglich.

Weiter umfasst die Messvorrichtung 1 eine Lichtquelle 7, beispielsweise eine UV-LED, zum Bestrahlen des Lipidsystems mit Licht bzw. zum Anregen eines in dem zu untersuchenden Lipidsystems enthaltenen fluoreszierenden Farbstoffs. Außerdem umfasst die Messvorrichtung 1 eine mit einem nicht gezeigten Detektor verbundene optische Faser 8 zum Erfassen (zumindest eines Teils) des von dem fluoreszierenden Farbstoff des Lipidsystems infolge der Anregung ausgesendeten Fluoreszenzspektrums. In der vorliegenden beispielhaft gezeigten Messvorrichtung 1 sind die Lichtquelle 7 und die optische Faser 8 senkrecht zueinander angeordnet, wobei auch andere Anordnungen denkbar sind. Wie in Fig. 1 veranschaulicht, liegt die optische Faser 8 außerhalb der dort gezeigten Schnittansicht. Zu Zwecken der Erläuterung der Messvorrichtung 1 ist die Schnittansicht aus Fig. 1 jedoch durch die außerhalb dieser Schnittansicht liegende optische Faser 8 ergänzt. Um eine Bestrahlen des Lipidsystems durch die Lichtquelle 7 sowie ein Erfassen des emittierten Fluoreszenzspektrums durch die optische Faser 8 zu ermöglichen, weist die Messvorrichtung 1, wie in Fig. 1 skizziert, entsprechende Öffnungen bzw. lichtdurchlässige Kanäle zwischen Lichtquelle 7 und der Glaskapillare 8 sowie zwischen der optischen Faser 8 und dem Probengefäß 6 auf.

In Fig. 2 und Fig. 3 ist beispielhaft das spektroskopische Ergebnis einer Messung eines mit einem fluoreszierenden Farbstoff versehenen zu untersuchenden Lipidsystems bei einer festen Lagertemperatur T sowie eine mögliche Auswertung gezeigt.

In Fig. 2 ist die erfasste Intensität der emittierten Strahlung gegenüber der Wellenlänge aufgetragen. Die Kreise symbolisieren dabei die einzelnen Messergebnisse und die durchgezogene Linie das Ergebnis eines Fits durch diese Messergebnisse. In dem gezeigten Beispiel waren charakteristische Spektrallinien bei den Wellenlängen λ₁ und λ₂ des verwendeten fluoreszierenden Farbstoffs bekannt. Hieraus wurden zunächst, wie in Fig. 2 durch die liniert eingezeichneten Flächen angedeutet, die Intensitäten für λ₁ und λ₂, d.h. I₁ und I₂, ermittelt. Aus diesen ermittelten Intensitätswerten wurde im Folgenden der Parameter P wie folgt berechnet: $P = \frac{{I}_{1} - {I}_{2}}{{I}_{1} + {I}_{2}}$

In Fig. 3 ist beispielhaft der Verlauf des P-Werts in Abhängigkeit der Lagerzeit gezeigt. Wie Fig. 3 anzeigt, ändert sich der P-Wert im Laufe der Lagerung, was auf eine Änderung des Lipidsystems zurückzuführen ist. Anstelle dem beispielhaft genannten Parameter P sind jedoch auch zahlreiche andere, aus den Messergebnissen ableitbare Größen denkbar, die zum Aufzeigen der Änderung des Lipidsystems geeignet sind.

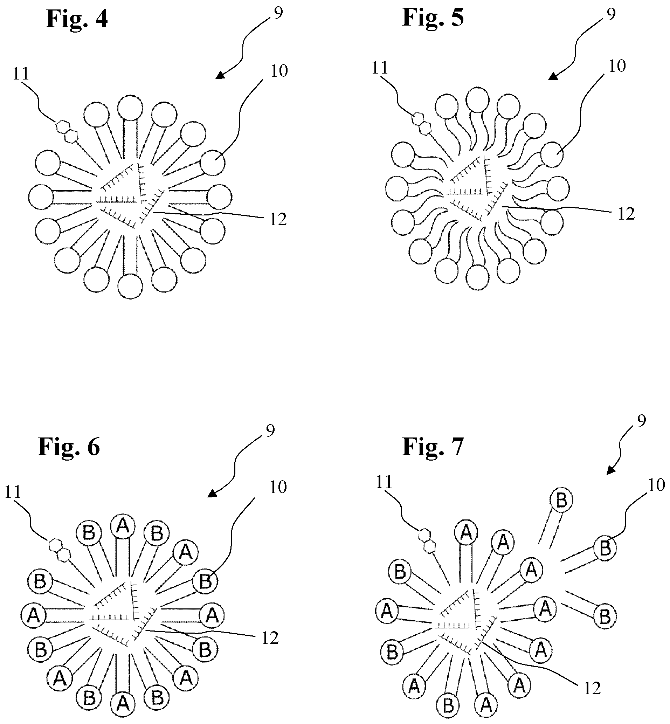

Exemplarisch sind in den Fig. 4 bis Fig. 7 Lipidsysteme 9 und denkbare Änderungen dieser Lipidsysteme 9 in Folge der Lagerung skizziert. Die beispielhaft skizzierten Lipidsysteme 9 umfassen dabei Lipide 10 mit jeweils einer hydrophilen Kopfgruppe und zwei lipophilen Kohlenwasserstoffketten. In den vorliegenden Beispielen bilden die Lipide 10 sphärische Strukturen in deren Inneren ein Wirkstoff 12 eingebettet ist. Weiter weisen die Lipidsysteme 9 einen fluoreszierender Farbstoff 11 auf, der Teil der sphärischen Struktur ist.

In Fig. 4 und Fig. 5 ist beispielhaft eine Änderung der Struktur bzw. Ordnung der Kohlenwasserstoffketten der Lipide 9 infolge der Lagerung skizziert. In Fig. 6 und Fig. 7 ist ein Lipidsystem mit den durch A und B gekennzeichneten unterschiedlichen Lipiden 9 und eine Veränderung der Komposition der sphärischen Lipidstrukturen infolge der Lagerung skizziert.

Neben den exemplarisch skizzierten Änderungen der Fig. 4 bis Fig. 7 sind auch zahlreiche weitere Änderungen eines Lipidsystems, wie beispielsweise die Änderung der Hydrierung, etc. möglich.

### Bezugszeichenliste

- 1: Messvorrichtung
- 2: Äußere Einheit
- 3: Mittlere Einheit
- 4: Innere Einheit
- 5: Temperatursensor
- 6: Probengefäß
- 7: Lichtquelle
- 8: Optische Faser
- 9: Lipidsystem
- 10: Lipide
- 11: Fluoreszierender Farbstoff
- 12: Wirkstoff

## Patentansprüche

1. Verfahren zur in-situ Erfassung von Änderungen der Struktur und/oder der Komposition und/oder der Hydrierung eines Lipidsystems (9) bei dessen Lagerung bei einer Lagertemperatur unterhalb von -60 °C umfassend:
• Bereitstellen des zu untersuchenden Lipidsystems (9) welches mit mindestens einem fluoreszierenden Farbstoff (11) versehen ist;
• Anregen des fluoreszierenden Farbstoffs (11) des Lipidsystems (9) mit Licht;
• Erfassen von zumindest einem Teil eines von dem fluoreszierenden Farbstoff (11) ausgesendeten Fluoreszenzspektrums mit einem Detektor;
• Vergleichen zumindest eines Teils des erfassten Fluoreszenzspektrums mit einem entsprechenden Teil eines Referenzspektrums; und
• Erfassen von Abweichungen gegenüber dem Referenzspektrum, die ein Maß für die Änderung des Lipidsystems (9) sind.

2. Verfahren nach einem der voranstehenden Ansprüche, **gekennzeichnet durch** das Erfassen und/oder Hinterlegen des Referenzspektrums, wobei das Referenzspektrum insbesondere ein erfasstes Fluoreszenzspektrum zu einem früheren Zeitpunkt der Lagerung des Lipidsystems (9), insbesondere zu einem Ausgangszeitpunkt bzw. zum Beginn der Lagerung, oder ein Fluoreszenzspektrum eines in einer Datenbank gespeicherten Referenzsystems ist.

3. Verfahren nach einem der voranstehenden Ansprüche, **gekennzeichnet durch** Vergleichen von Intensitätswerten einer oder mehrerer einzelner Wellenlängen oder einzelner Wellenlängenbereiche des erfassten Fluoreszenzspektrums mit entsprechenden Intensitätswerten der Wellenlängen oder Wellenlängenbereiche des Referenzspektrums.

4. Verfahren nach einem der voranstehenden Ansprüche, **gekennzeichnet durch** das Erfassen einer vorgegebenen Polarisation des ausgesendeten Fluoreszenzspektrums und/oder das Vergleichen der Polarisation des ausgesendeten Fluoreszenzspektrums mit einer Polarisation des Referenzspektrums.

5. Verfahren nach einem der voranstehenden Ansprüche, **dadurch gekennzeichnet, dass** das Vergleichen zumindest eines Teils des erfassten Fluoreszenzspektrums mit einem entsprechenden Teil eines Referenzspektrums auf Basis von aus den genannten Spektren berechneten Parametern erfolgt.

6. Verfahren nach einem der voranstehenden Ansprüche, **gekennzeichnet durch** eine Erfassung der Änderungen des Lipidsystems (9) in Abhängigkeit der Lagerzeit und/odereine Erfassung der Änderungen des Lipidsystems (9) in Abhängigkeit der Lagertemperatur.

7. Verfahren nach einem der voranstehenden Ansprüche, **gekennzeichnet durch** eine kurzzeitige Änderung der Lagertemperatur und Erfassen der Änderung des Lipidsystems (9) in Abhängigkeit einer kurzzeitigen Änderung der Lagertemperatur und/oderdas Erfassen der temperaturprofilabhängigen Änderung des Lipidsystems (9) bei der anfänglichen Abkühlung des Lipidsystems (9) auf die Lagertemperatur und/oder bei abschließender Erwärmung des Lipidsystems (9) nach erfolgter Lagerung auf eine Gebrauchstemperatur, insbesondere bei Erwärmen auf eine Temperatur über 0°C.

8. Verfahren nach einem der voranstehenden Ansprüche **gekennzeichnet durch** einen Schritt des Bestimmens von geeigneten Lagerbedingungen des Lipidsystems (9), insbesondere einer geeigneten Lagertemperatur und/oder Lagerzeit, bei denen eine durch die Fluoreszenzspektroskopie erfassbare Änderung des Lipidsystems (9) unterhalb eines vorgegebenen Grenzwerts liegt.

9. Verfahren nach einem der voranstehenden Ansprüche, **gekennzeichnet durch** Ausgabe eines Warnsignals und/oder eines Warnhinweises bei Erfassen einer Abweichung des Fluoreszenzspektrums gegenüber dem Referenzspektrum, die über eine vorgegebene Grenzabweichung hinausgeht und/oder ein Anpassen der Lagertemperatur während der Lagerung infolge der erfassten Abweichung.

10. Verfahren nach einem der voranstehenden Ansprüche, **gekennzeichnet durch** einen Schritt des Bestimmens und/oder der Charakterisierung der Änderung der Struktur und/oder der Komposition und/oder der Hydrierung auf Basis der erfassten Abweichungen und/oder einen Schritt des Vergleichens einer erfassten Änderung des Lipidsystems (9) mit der durch ein anderes Messverfahren erfassten Änderung, insbesondere mit der durch eine kalorimetrische Messung und/oder mittels dynamischer Lichtstreuung an dem Lipidsystem (9) bei Temperaturen oberhalb von 0° C erfassten Änderung.

11. Verfahren nach einem der voranstehenden Ansprüche, **dadurch gekennzeichnet, dass** es sich bei dem Lipidsystem (9) um ein System umfassend kollodiale Partikel, insbesondere Lipidnanopartikel, handelt und/oder dass das Lipidsystem (9) Träger eines Pharmazeutikums, insbesondere eines nukleinsäurebasierten Wirk- oder Impfstoffs (12), ist.

12. Verfahren nach einem der voranstehenden Ansprüche, **dadurch gekennzeichnet, dass** es sich bei dem fluoreszierenden Farbstoff (11) um Laurdan (C₂₄H₃₅NO), Prodan (C₁₅H₁₇NO), C-Laurdan (C₂₅H₃₅NO₃), Pro12A (C₃₁H₄₇N₂O₅SNa₂), Di-4-ANEPPDHQ (C₃₂H₄₇Br₂N₃O₂) oder Atto 488 mit verschiedenen Lipidankernen oder einer Kombination dieser handelt.

13. Verfahren nach einem der voranstehenden Ansprüche, **dadurch gekennzeichnet, dass** die Lagertemperatur unterhalb von -80°C, insbesondere unterhalb von -100 °C, ist.

14. Verfahren nach einem der voranstehenden Ansprüche, **dadurch gekennzeichnet, dass** das Licht zum Anregen des fluoreszierenden Farbstoffs (11), Licht im UV-Bereich und/oder im sichtbaren- Bereich und/oder IR-Bereich ist.

15. Verfahren nach einem der voranstehenden Ansprüche, **gekennzeichnet durch** ein Speichern des erfassten Fluoreszenzspektrums und/oder hieraus berechneter Parameter und/oder von Änderungen des Lipidsystems, insbesondere in Abhängigkeit der Lagerzeit und/oder der Lagertemperatur, in einer Datenbank.

## Claims

1. Method for in-situ detection of changes in the structure and/or composition and/or hydrogenation of a lipid system (9) during the storage thereof at a storage temperature below -60°C, comprising
• Preparing the lipid system (9) being investigated, which is provided with at least one fluorescent dye (11);
• Exciting the fluorescent dye (11) of the lipid system (9) with light;
• Detecting at least part of the fluorescence spectrum emitted by the fluorescent dye (11) with a detector;
• Comparing at least part of the detected fluorescence spectrum with a corresponding part of a reference spectrum; and
• Detecting deviations relative to the reference spectrum, which are a measure of the change in the lipid system (9).

2. Method according to one of the preceding claims, **characterized by** detecting and/or storing the reference spectrum, wherein the reference spectrum is, in particular, a recorded fluorescence spectrum at an earlier time of storage of the lipid system (9), in particular at an initial time or at the beginning of storage, or a fluorescence spectrum of a reference system stored in a database.

3. Method according to one of the preceding claims, **characterized by** comparing intensity values of one or more individual wavelengths or individual wavelength regions of the detected fluorescence spectrum with corresponding intensity values of the wavelengths or wavelength regions of the reference spectrum.

4. Method according to one of the preceding claims, **characterized by** detecting a stipulated polarization of the emitted fluorescence spectrum and/or comparing the polarization of the emitted fluorescence spectrum with a polarization of the reference spectrum.

5. Method according to one of the preceding claims, **characterized in that** comparison of at least a part of the detected fluorescence spectrum with a corresponding part of the reference spectrum occurs based on parameters calculated from the aforementioned spectra.

6. Method according to one of the preceding claims, **characterized by** detecting the changes in the lipid system (9) as a function of storage time and/or detecting the changes in the lipid system (9) as a function of storage temperature.

7. Method according to one of the preceding claims, **characterized by** a brief change in the storage temperature and detection of the change in the lipid system (9) as a function of the brief change in the storage temperature and/or by detecting the temperature profile-dependent change in the lipid system (9) during initial cooling of the lipid system (9) to the storage temperature and/or upon final heating of the lipid system (9) after storage to a use temperature, in particular upon heating to a temperature above 0°C.

8. Method according to one of the preceding claims, **characterized by** a step of determining appropriate storage conditions of the lipid system (9), in particular an appropriate storage temperature and/or storage time at which a change in the lipid system (9) detectable by fluorescence spectroscopy lies below a stipulated limit value.

9. Method according to one of the preceding claims, **characterized by** issuing a warning signal and/or a warning instruction when a deviation is detected in the fluorescence spectrum relative to the reference spectrum which exceeds a stipulated limit deviation and/or by adjusting the storage temperature during storage as a result of the detected deviation.

10. Method according to one of the preceding claims, **characterized by** a step of determining and/or characterizing the change in the structure and/or composition and/or hydrogenation based on the detected deviation and/or by a step of comparing a detected change in the lipid system (9) with the change detected by another measurement method, in particular the change detected by a calorimetric measurement and/or by dynamic light scattering of the lipid system (9) at temperatures above 0°C.

11. Method according to one of the preceding claims, **characterized in that** the lipid system (9) is a system comprising colloidal particles, in particular lipid nanoparticles and/or **in that** the lipid system (9) is a carrier of a pharmaceutical, in particular a nucleic acid-based active ingredient or vaccine (12).

12. Method according to the one of preceding claims, **characterized in that** the fluorescent dye (11) is Laurdan (C₂₄H₃₅NO), Prodan (C₁₅H₁₇NO), C-Laurdan (C₂₅H₃₅NO₃), Pro12A (C₃₁H₄₇N₂O₅SNa₂), Di-4-ANEPPDHQ (C₃₂H₄₇Br₂N₃O₂) or Atto 488 with different lipid anchors or a combination thereof.

13. Method according to one of the preceding claims, **characterized in that** the storage temperature is below -80°C, in particular below -100°C.

14. Method according to one of the preceding claims, **characterized in that** the light for exciting fluorescent dye (11) is light in the UV range and/or visible range and/or IR range.

15. Method according to one of the preceding claims, **characterized by** storing in a database the detected fluorescence spectrum and/or parameters calculated therefrom and/or changes in the lipid system, in particular as a function of the storage time and/or the storage temperature.

## Revendications

1. Procédé pour la détection in situ de modifications de la structure et/ou de la composition et/ou de l'hydrogénation d'un système lipidique (9) lors de son stockage à une température de stockage inférieure à -60 °C, comprenant :
• la fourniture du système lipidique (9) à analyser, lequel est pourvu d'au moins un colorant fluorescent (11) ;
• l'excitation par la lumière du colorant fluorescent (11) du système lipidique (9) ;
• la détection, au moyen d'un détecteur, d'au moins une partie d'un spectre de fluorescence émis par le colorant fluorescent (11) ;
• la comparaison d'au moins une partie du spectre de fluorescence détecté avec une partie correspondante d'un spectre de référence ; et
• la détection d'écarts par rapport au spectre de référence, lesquels constituent une mesure de la modification du système lipidique (9).

2. Procédé selon l'une des revendications précédentes, **caractérisé par** la détection et/ou l'enregistrement du spectre de référence, dans lequel le spectre de référence est en particulier un spectre de fluorescence détecté à un moment antérieur du stockage du système lipidique (9), en particulier à un moment initial ou au début du stockage, ou un spectre de fluorescence d'un système de référence mémorisé dans une base de données.

3. Procédé selon l'une des revendications précédentes, **caractérisé par** une comparaison de valeurs d'intensité d'une ou plusieurs longueurs d'onde individuelles ou de gammes de longueurs d'onde individuelles du spectre de fluorescence détecté avec des valeurs d'intensité correspondantes des longueurs d'onde ou des gammes de longueurs d'onde du spectre de référence.

4. Procédé selon l'une des revendications précédentes, **caractérisé par** la détection d'une polarisation prédéfinie du spectre de fluorescence émis et/ou la comparaison de la polarisation du spectre de fluorescence émis avec une polarisation du spectre de référence.

5. Procédé selon l'une des revendications précédentes,
**caractérisé en ce que** la comparaison d'au moins une partie du spectre de fluorescence détecté avec une partie correspondante d'un spectre de référence est effectuée sur la base de paramètres calculés à partir desdits spectres.

6. Procédé selon l'une des revendications précédentes, **caractérisé par** une détection des modifications du système lipidique (9) en fonction de la durée de stockage et/ou une détection des modifications du système lipidique (9) en fonction de la température de stockage.

7. Procédé selon l'une des revendications précédentes, **caractérisé par** une modification de courte durée de la température de stockage et une détection de la modification du système lipidique (9) en fonction d'une modification de courte durée de la température de stockage et/ou la détection de la modification dépendant du profil de température du système lipidique (9) lors du refroidissement initial du système lipidique (9) à la température de stockage et/ou lors d'un réchauffement final du système lipidique (9) après un stockage effectué à une température d'utilisation, en particulier lors d'un réchauffement à une température supérieure à 0 °C.

8. Procédé selon l'une des revendications précédentes, **caractérisé par** une étape de détermination de conditions de stockage appropriées du système lipidique (9), en particulier d'une température de stockage et/ou d'une durée de stockage appropriées, dans lesquelles une modification du système lipidique (9) détectable par spectroscopie de fluorescence est inférieure à une valeur limite prédéfinie.

9. Procédé selon l'une des revendications précédentes, **caractérisé par** une émission d'un signal d'alerte et/ou d'une indication d'avertissement lors d'une détection d'un écart du spectre de fluorescence par rapport au spectre de référence, lequel écart dépasse un écart limite prédéfini, et/ou un ajustement de la température de stockage pendant le stockage à la suite de l'écart détecté.

10. Procédé selon l'une des revendications précédentes, **caractérisé par** une étape de détermination et/ou de caractérisation de la modification de la structure et/ou de la composition et/ou de l'hydrogénation sur la base des écarts détectés et/ou une étape de comparaison d'une modification détectée du système lipidique (9) avec la modification détectée par un autre procédé de mesure, en particulier avec la modification détectée par une mesure calorimétrique et/ou au moyen d'une dispersion dynamique de la lumière sur le système lipidique (9) à des températures supérieures à 0 °C.

11. Procédé selon l'une des revendications précédentes,
**caractérisé en ce que** le système lipidique (9) est un système comprenant des particules colloïdales, en particulier des nanoparticules lipidiques, **et/ou en ce que** le système lipidique (9) est un support d'un produit pharmaceutique, en particulier d'une substance active ou d'un vaccin (12) à base d'acide nucléique.

12. Procédé selon l'une des revendications précédentes,
**caractérisé en ce que** le colorant fluorescent (11) est du Laurdan (C₂₄H₃₅NO), Prodan (C₁₅H₁₇NO), C-Laurdan (C₂₅H₃₅NO₃), Pro12A (C₃₁H₄₇N₂O₅SNa₂), Di-4-ANEPPDHQ (C₃₂H₄₇Br₂N₃O₂) ou Atto 488 comportant différents ancrages lipidiques, ou une combinaison de ceux-ci.

13. Procédé selon l'une des revendications précédentes,
**caractérisé en ce que** la température de stockage est inférieure à -80 °C, en particulier inférieure à -100 °C.

14. Procédé selon l'une des revendications précédentes,
**caractérisé en ce que** la lumière pour l'excitation du colorant fluorescent (11) est une lumière dans la gamme des UV et/ou dans la gamme du visible et/ou dans la gamme des infrarouges.

15. Procédé selon l'une des revendications précédentes, **caractérisé par** une mémorisation, dans une base de données, du spectre de fluorescence détecté et/ou de paramètres calculés à partir de celui-ci et/ou de modifications du système lipidique, en particulier en fonction de la durée de stockage et/ou de la température de stockage.
